# EUROPÄISCHE PATENTANMELDUNG

(11) **EP 0 586 875 A1**
(43) Veröffentlichungstag der Anmeldung: **16.03.1994**
(21) Anmeldenummer: 93112378.0
(22) Anmeldetag: 01.02.1989
(51) Int. Cl.: A61K 49/00

(54) **Ultraschallkontrastmittel, Verfahren zu deren Herstellung und deren Verwendung als Diagnostika und Therapeutika**

(30) Priorität: 05.02.1988 DE 3803972; 05.02.1988 DE 3803971
(62) Teilanmeldung aus: 89901933.5
(71) Anmelder: SCHERING AKTIENGESELLSCHAFT, D-13353 Berlin (DE)
(72) Erfinder: Stein, Michael, Dr., D-10713 Berlin (DE); Heldmann, Dieter, Dr., D-10555 Berlin (DE); Fritzsch, Thomas, Dr., D-12169 Berlin (DE); Rössling, Georg, Dr., D-13465 Berlin (DE); Siegert, Joachim, Dr., D-12157 Berlin (DE); Speck, Ulrich, Prof., D-13465 Berlin (DE)

(57) **Zusammenfassung**

Die Erfindung betrifft gas- bzw. flüssigkeitshaltige Mikropartikel auf der Basis von Amylosen, Verfahren zu deren Herstellung und deren Verwendung als Diagnostika.

## Beschreibung

Die Erfindung betrifft Mikropartikel nach dem Oberbegriff des Patentanspruchs 1, Verfahren zu deren Herstellung und deren Verwendung als Diagnostika und Therapeutika.

Es ist bekannt, daß durch periphere Injektion von Lösungen, die feine Gasblasen enthalten, cardiale Echokontraste erzielt werden können [Roelandt J, Ultrasound Med. Biol. 8: 471-492, (1982)]. Diese Gasblasen werden in physiologisch verträglichen Lösungen, z.B. durch Schütteln, andere Agitation oder durch Zusatz von Kohlendioxid erhalten. Sie sind jedoch hinsichtlich Anzahl und Größe nicht standardisiert und können nur unzulänglich reproduziert werden. Auch sind sie in der Regel nicht stabilisiert, so daß ihre Lebensdauer gering ist. Ihre mittleren Durchmesser liegen meist über Erythrocytengröße, so daß keine Lungenkapillarpassage mit nachfolgender Kontrastierung von Organen wie linkes Herz, Leber, Niere oder Milz möglich ist. Darüber hinaus eignen sie sich nicht für Quantifizierungen, da sich das von ihnen erzeugte Ultraschallecho aus mehreren, nicht voneinander zu trennenden Prozessen wie Blasenentstehung, Koaleszenz und Auflösung zusammensetzt. So ist es z.B. nicht möglich, mit Hilfe dieser Ultraschall-Kontrastmittel über die Messung des Kontrastverlaufs im Myokard Aussagen über die Transitzeiten zu gewinnen. Hierzu sind Kontrastmittel notwendig, deren Streukörper keiner eigenen Kinetik unterliegen.

Daneben gibt es Ultraschallkontrastmittel in Form von Partikeln [Ophir, Gobuty, McWhirt, Maklad, Ultrasonic Backscatter from Contrast-producing Collagen Microspheres, Ultrasonic Imaging 2: 66-67, (1980)]. Ferner werden [Ophir, McWhirt, Maklad, Aqueous Solutions as Potential Ultrasonic Contrast Agents, Ultrasonic Imaging 1: 265-279, (1979) sowie Tyler, Ophir, Maklad, In-vivo Enhancement of Ultrasonic Image Luminance by Aqueous Solutions with High Speed of Sound, Ultrasonic Imaging 3: 323-329, (1981)] Lösungen höherer Dichte als Ultraschall-Kontrastmittel eingesetzt. Es ist auch bekannt, Emulsionen als Ultraschall-Kontrastmittel zu verwenden [Mattrey, Andre, Ultrasonic Enhancement of Myocardial Infarction with Perfluorcarbon Compounds in Dogs, Am. J. Cardiol 54: 206-210, (1984)].

Es hat sich gezeigt, daß die gasfreien Kontrastmittel insgesamt nur eine geringe Effizienz besitzen. Die gashaltigen Zubereitungen haben den Nachteil einer nur geringen in-vivo Stabilität. Darüber hinaus ist die Größe der Gasblasen meistens nicht standardisierbar. Ausreichende Kontrasteffekte sind im arteriellen Gefäßsystem nach peripher venöser Injektion in aller Regel nicht möglich.

In den EP-A 2 123 235 und 0 122 624 werden Gasbläschen enthaltende Ultraschall-Kontrastmittel beschrieben, die die Lungenkapillaren passieren können und damit den gewünschten Kontrasteffekt bewirken.

Die EP-A2 0 224 934 beschreibt Ultraschall-Kontrastmittel in Form von gasgefüllten Gelatine- oder Albuminhohlkörpern. Nachteilig ist jedoch die Verwendung von körper-fremden oder denaturierten körpereigenen Eiweißen wegen des damit verbundenen allergenen Risikos.

Mit keinem der bisher bekannten Ultraschall-Kontrastmittel gelingt eine Organdarstellung mit ausreichender Signalintensität durch selektive Anreicherung nach i.v. Gabe. Quantifizierungen sind daher z.Zt. nicht möglich.

Der Erfindung liegt die Aufgabe zugrunde, Ultraschall-Kontrastmittel auf der Basis von Mikropartikeln herzustellen, die neben bestimm- und reproduzierbaren Volumina eine erheblich längere Lebensdauer als bisher bekannt aufweisen, gute Verträglichkeit ohne allergenes Potential besitzen und intrazellulär in RES und damit auch in der Leber oder Milz angereichert werden können.

Erfindungsgemäß wird diese Aufgabe durch Mikropartikel, die aus Amylosen und einem Gas und/oder einer Flüssigkeit mit einem Siedepunkt unter 60 °C bestehen, gelöst.

Stärke oder Stärkederivate können ebenso in den Mikropartikeln enthalten sein. Als geeignet haben sich Amylosen erwiesen, da sich diese Stärkederivate durch gute Wasser-löslichkeit und der Fähigkeit zur Bildung von Einschlußverbindungen auszeichnen.

Als besonders geeignete Amylosen sind die Cyclodextrine und deren Derivate, beispiels-weise α-, β-, und γ-Cyclodextrin zu nennen.

Die Mikropartikel enthalten Gase und/oder Flüssigkeiten mit einem Siedepunkt unter 60 °C in freier oder gebundener Form. Die Verwendung eines Gas-Flüssigkeits-Gemisches in den Ultraschall-Kontrastmitteln ist ebenfalls möglich.

Als Gase können beispielsweise Luft, Stickstoff, Edelgase, Wasserstoff, Kohlendioxid, Ammoniak, Sauerstoff, Methan, Ethan, Propan, Butan, Ethylen oder andere Kohlenwasserstoffe oder deren Gemische verwendet werden.

Als einschließbare Flüssigkeiten werden bevorzugt 1.1-Dichlorethylen, 2-Methyl-2-buten, Isopropylchlorid, 2-Methyl-1.3-butadien, 2-Butin, 2-Methyl-1-buten, Dibromdifluormethan, Furan, 3-Methyl-1-buten, Isopentan, Diethylether, 3.3-Dimethyl-1-butin, Dimethylaminoaceton, Propylenoxid, N-Ethylmethylamin, Brommethan, N-Ethyldimethylamin, Methylenchlorid, Pentan, Cyclopentan, 2,3-Pentaiden, Cyclopenten oder deren Gemische verwendet.

Mit Vorteil können die Mikropartikel auch Substanzen mit niedrigen Dampfdrücken und/oder niedrigen Siedepunkten, insbesondere ätherische Öle enthalten.

Besonders vorteilhaft ist es, die aus Amylosen bestehenden Mikropartikel mit einer Hüllsubstanz zu überziehen. Dabei können die Mikropartikel von Ölen, Fetten und/oder oberflächenaktiven Substanzen umhüllt und in wäßrigem Medium suspendiert sein.

Besonders vorteilhaft ist es, die aus Amylosen bestehenden Mikropartikel mit einer Matrix, insbesondere polymerer Struktur zu umhüllen.

Durch Zugabe osmotisch aktiver Substanzen, beispielsweise Kochsalz, Galaktose, Glukose, Fruktose, kann die physiologische Isotonie eingestellt werden.

Die vorstehend beschriebenen Ultraschall-Kontrastmittel können sowohl für diagnostische als auch für therapeutische Verfahren verwendet werden.

Die Applikation der Mittel erfolgt beispielsweise durch Injektionen.

Die Erfindung wird durch folgende Beispiele erläutert:

### Beispiel 1

### β-Cyclodextrin-Isopentan-Einschlußverbindung

100 ml gesättigte β-Cyclodextrin-Lösung (1,8 %ig) wird auf 10 °C gekühlt und mit 3 ml Isopentan versetzt. Unter ständigem Rühren im Ultraschallbad wird der entstehende schwerlösliche Komplex ausgefällt. Durch Gefriertrocknen und Filtrieren wird der Nieder-schlag in kristalliner Form erhalten. Isopentangehalt nach GC-Bestimmung: 0,26 %.

### Beispiel 2

### β-Cyclodextrin-2-Methyl-2-buten-Einschlußverbindung

100 ml gesättigte β-Cyclodextrin-Lösung (1,8 %ig) wird auf 10 °C gekühlt und mit 3 ml 2-Methyl-2-buten versetzt. Unter ständigem Rühren im Ultraschallbad wird der entste-hende schwerlösliche Komplex ausgefällt. Durch Gefriertrocknen und Filtrieren wird der Niederschlag in kristalliner Form erhalten.

### Beispiel 3

### β-Cyclodextrin-2-Methyl-1-buten-Einschlußverbindung

100 ml gesättigte β-Cyclodextrin-Lösung (1,8 %ig) wird auf 10 °C gekühlt und mit 3 ml 2-Methyl-1-buten versetzt. Unter ständigem Rühren im Ultraschallbad wird der entste-hende schwerlösliche Komplex ausgefällt. Durch Gefriergetrocknen und Filtrieren wird der Niederschlag in kristalliner Form erhalten. 2-Methyl-1-buten-Gehalt nach GC-Bestimmung: 0,82 %.

### Beispiel 4

### β-Cyclodextrin-Isopren-Einschlußverbindung

100 ml gesättigte β-Cyclodextrin-Lösung (1,8 %ig) wird auf 10 °C gekühlt und mit 3 ml Isopren versetzt. Unter ständigem Rühren im Ultraschallbad wird der entstehende schwer-lösliche Komplex ausgefüllt. Durch Gefriergetrocknen und Filtrieren wird der Nieder-schlag in kristalliner Form erhalten. Isoprengehalt nach GC-Bestimmung: 1,0 %.

### Beispiel 5

### β-Cyclodextrin-Isopropylchlorid-Einschlußverbindung

100 ml gesättigte β-Cyclodextrin-Lösung (1,8 %ig) wird auf 10 °C gekühlt und mit 3 ml Isopropylchlorid versetzt. Unter ständigem Rühren im Ultraschallbad wird der entstehende schwerlösliche Komplex ausgefällt. Durch Gefriertrocknen und Filtrieren wird der Nieder-schlag in kristalliner Form erhalten. Isopropylchloridgehalte nach GC-Bestimmung: 0,5 %.

### Beispiel 6

### β-Cyclodextrin-Isopentan-Einschlußverbindung

100 ml gesättigte β-Cyclodextrin-Lösung (1,8 %ig) wird auf 10 °C gekühlt und mit 3 ml Isopentan versetzt. Unter ständigem Rühren im Ultraschallbad wird der entstehende schwerlösliche Komplex ausgefällt. Durch Gefriertrocknen und Filtrieren wird der Nieder-schlag in kristalliner Form erhalten.

### Beispiel 7

### Xenon/α-Cyclodextrin-Einschlußverbindungen

In einem 200-cm³-Autoklaven werden 100 ml gesättigte α-Cyclodextrin-Lösung (ca. 12 %ig) unter 7 Atmosphären Xenon 7 Tage lang bei Zimmertemperatur inkubiert. Das kristalline Addukt kann abgesaugt, mit kaltem Wasser gewaschen und im Exsikkator über Calciumchlorid getrocknet werden.

### Beispiel 8

### Kohlendioxid/α-Cyclodextrin-Einschlußverbindung

In einem 200-cm³-Autoklaven werden 100 ml gesättigte α-Cyclodextrin-Lösung (ca. 12 %ig unter 7 Atmosphären Kohlendioxid 7 Tage lang bei Zimmertemperatur inkubiert. Das kristalline Addukt kann abgesaugt, mit kaltem Wasser gewaschen und im Exsikkator über Calciumchlorid getrocknet werden.

### Beispiel 9

### Isopentan/Hydroxypropyl-β-Cyclodextrin-Einschlußverbindung

15 ml 20 %ige Hydroxypropyl-β-Cyclodextrin-Lösung werden bei 10 °C mit 2 ml Isopentan versetzt, im Ultraschallbad 3 Minuten beschallt und danach für 26 Stunden inkubiert. Der entstandene Komplex bleibt in Lösung.

### Beispiel 10

### Isopren/Hydroxypropyl-β-Cyclodextrin-Einschlußverbindung

15 ml 20 %ige Hydroxypropyl-β-Cyclodextrin-Lösung werden bei 10 °C mit 2 ml Isopren versetzt, im Ultraschallbad 3 Minuten beschallt und danach für 26 Stunden inkubiert. Der entstandene Komplex bleibt zum Teil in Lösung und fällt zu einem Teil als weißer Nieder-schlag aus.

### Beispiel 11

### Furan/Hydroxypropyl-β-Cyclodextrin-Einschlußverbindung

15 ml 20 %ige Hydroxypropyl-β-Cyclodextrin-Lösung werden bei 10 °C mit 2 ml Furan versetzt, im Ultraschallbad 3 Minuten beschallt und danach für 26 Stunden inkubiert. Der entstandene Komplex bleibt zum Teil in Lösung und fällt zu einem Teil als weißer Nieder-schlag aus.

### Beispiel 12

### Isopentan/α-Cyclodextrin-Einschlußverbindung

20 ml gesättigte α-Cyclodextrin-Lösung werden mit 1 ml Isopentan versetzt und im Ultra-schallbad 3 Minuten beschallt. Der entstehende schwerlösliche Komplex wird durch Filtration gewonnen und über Calciumchlorid getrocknet.

### Beispiel 13

### Isopren/α-Cyclodextrin-Einschlußverbindung

20 ml gesättigte α-CD-Lösung werden mit 1 ml Isopren versetzt und im Ultraschallbad 3 Minuten beschallt. Der entstehende schwerlösliche Komplex wird durch Filtration gewonnen und über Calciumchlorid getrocknet.

### Beispiel 14

### Furan/α-Cyclodextrin-Einschlußverbindung

20 ml gesättigte α-Cyclodextrin-Lösung werden mit 1 ml Furan versetzt und im Ultraschallbad 3 Minuten beschallt. Der entstehende schwerlösliche Komplex wird durch Filtration gewonnen und über Calciumchlorid getrocknet.

### Beispiel 15

In einem Inkubationsgefäß werden zu 100 ml ges. α-Cyclodextrin-Lösung (5 °C) unter Beschallung im Ultraschallbad 4 g Eukalyptol zugetropft und für weitere 30 Minuten beschallt. Danach wird der Ansatz 48 Stunden in einem gekühlten, geschlossenen Gefäß geschüttelt. Der entstandene Niederschlag wird durch Filtration gewonnen, mit kaltem Ethanol gewaschen, in fl. N₂ eingefroren und gefriergetrocknet.

### Beispiel 16

100 ml ges. β-Cyclodextrin-Lösung werden mit 2 g Geraniol bei 5 °C 4 Stunden im Ultra-schallbad beschallt und danach für 24 Stunden bei 5 °C inkubiert. Der entstandene Niederschlag wird abfiltriert, mit kaltem Ethanol gewaschen, in fl. N₂ eingefroren und gefrier-getrocknet.

### Für die Beispiele 11 bis 16 gilt:

Der kristalline Niederschlag wird nach dem Reinigen in einem geeigneten wäßrigen Medium, vorzugsweise physiologischer Kochsalz-, Glukose- oder Ringerlösung aufgenommen und ist dann injektionsfähig.

## Patentansprüche

1. Ultraschall-Kontrastmittel bestehend aus Mikropartikeln, dadurch gekennzeichnet, daß die Mikropartikel aus Amylosen und einem Gas und/oder einer organischen Flüssigkeit mit einem Siedepunkt unter 60 °C bestehen.

2. Ultraschall-Kontrastmittel nach Anspruch 1, dadurch gekennzeichnet, daß die Mikropartikel als Amylosen Cyclodextrine oder Cyclodextrinderivate enthalten.

3. Ultraschall-Kontrastmittel nach mindestens einem der Ansprüche 1 bis 2, dadurch gekennzeichnet, daß die Mikropartikel als organische Flüssigkeiten mit einem Siedepunkt unter 60 °C 1.1-Dichlorethylen, 2-Methyl-2-buten, Isopropylchlorid, 2-Methyl-1.3-butadien, 2-Butin, 2-Methyl-1-buten, Dibromdifluormethan, Furan, 3-Methyl-1-buten, Isopentan, Diethylether, 3.3-Dimethyl-1-butin, Dimethylaminoaceton, Propylenoxid, N-Ethylmethylamin, Brommethan, N-Ethyldimethylamin, Methylenchlorid, Pentan, Cyclopentan, 2,3-Pentaiden, Cyclopenten oder deren Gemische enthalten.

4. Ultraschall-Kontrastmittel nach mindestens einem der Ansprüche 1 bis 2, dadurch gekennzeichnet, daß die Mikropartikel als Gase Luft, Edelgase Stickstoff, Sauerstoff, Kohlendioxid, Wasserstoff, Ammoniak, Ethylen, Methan, Ethan, Propan oder Butan oder deren Gemische enthalten.

5. Ultraschall-Kontrastmittel nach Anspruch 1, dadurch gekennzeichnet, daß die Mikropartikel ätherische Öle enthalten.

6. Ultraschall-Kontrastmittel nach mindestens einem der Ansprüche 1 bis 5, dadurch gekennzeichnet, daß die aus Amylosen bestehenden Mikropartikel mit einer hydrophoben Hüllsubstanz bestehend aus Ölen, Fetten und/oder oberflächenaktiven Substanzen überzogen und in wäßrigem Medium suspendiert sind.

7. Ultraschall-Kontrastmittel nach mindestens einem der Ansprüche 1 bis 6, dadurch gekennzeichnet, daß die aus Amylosen bestehenden Mikropartikel von einer Matrix, insbesondere polymerer Struktur, umhüllt sind.

8. Ultraschall-Kontrastmittel nach mindestens einem der Ansprüche 1 bis 7, dadurch gekennzeichnet, daß durch Zugabe osmotisch aktiver Substanzen, insbesondere Kochsalz, Mannit, Galaktose, Glukose, Fruktose, die physiolgische Isotonie eingestellt ist.

9. Verwendung von Mikropartikeln gemäß den Ansprüchen 1 bis 8 enthaltend ein Gas oder eine organische Flüssigkeit und Amylosen zur Herstellung eines Präparates für die Ultraschalldiagnostik oder -therapie.
